# EUROPEAN PATENT SPECIFICATION

(11) **EP 2 658 839 B1**
(45) Date of publication and mention of the grant of the patent: **22.10.2014**
(21) Application number: 11817220.4
(22) Date of filing: 28.12.2011
(51) Int. Cl.: C07C 201/02

(54) **PROCESS FOR THE MANUFACTURE OF NICORANDIL**
VERFAHREN ZUR HERSTELLUNG VON NICORANDIL
PROCÉDÉ DE FABRICATION DE NICORANDIL

(30) Priority: 28.12.2010 IT MI20102419
(43) Date of publication of application: 06.11.2013
(73) Proprietor: Procos S.p.A., 28062 Cameri NO (IT)
(72) Inventor: BAROZZA, Alessandro, I-20020 Nosate (MI) (IT); COLOMBO, Matteo, I-28062 Cameri (NO) (IT); ROLETTO, Jacopo, I-10135 Torino (IT); PAISSONI, Paolo, I-10040 Druento (TO) (IT)
(74) Representative: Minoja, Fabrizio
(86) International application number: PCT/EP2011/074152
(87) International publication number: WO 2012/089769

(56) References cited:
- US-A- 4 200 640
- EREMENKO, L. T.; NESTERENKO, D. A.; GARANIN, V. A.; KOSILKO, V. P.: "Development of improved laboratory technique for nicorandil synthesis", RUSSIAN JOURNAL OF APPLIED CHEMISTRY, vol. 82, 2009, pages 1776-1779, XP002652992, DOI: 10.1134/S107042720910005X cited in the application

## Description

### SUMMARY OF THE INVENTION

The present invention relates to a process for the synthesis of Nicorandil **(1),** 2-(nicotinamide)ethyl nitrate starting from N-(2-hydroxyethyl)nicotinamide **(15),** using nitration with nitric acid in the presence of acetic anhydride.

### BACKGROUND TO THE INVENTION

Nicorandil is a vasodilator used to treat angina pectoris. A number of synthesis methods are known.

DE 2714713 discloses the preparation of Nicorandil (1) starting from nicotinoyl chloride hydrochloride (2) and the nitric salt of 2-aminoethyl nitrate **(3)**

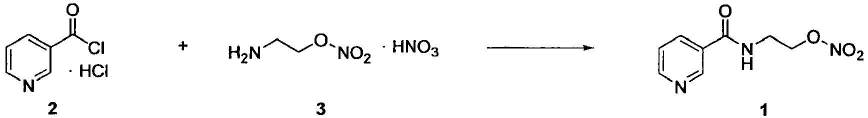

A slightly modified version of the scheme proposed in DE 2714713 is used in various references. US 4200640 discloses the preparation of Nicorandil starting from nicotinoyl chloride hydrochloride **(2)** and 2-aminoethyl nitrate **(4)** in the presence of pyridine

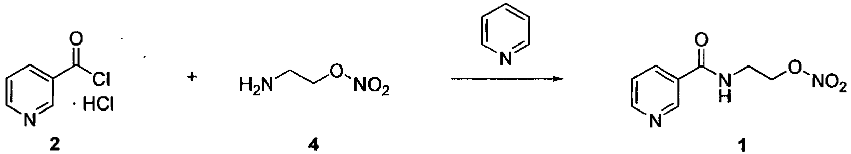

In the *"*Journal of Pharmaceutical Sciences (1999), 61(5), 304-305 *"* the reaction is effected starting from nicotinoyl chloride (5), which is treated with 2-aminoethyl nitrate **(4),** again in the presence of pyridine 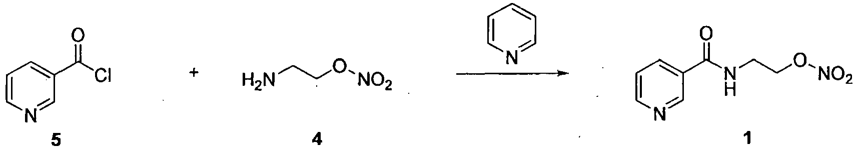 while IN178880 conducts the synthesis with nicotinic acid methyl ester (6)

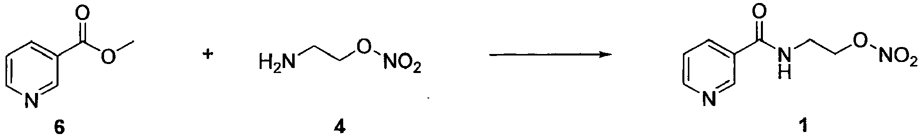

In *"*Rev Med Chir Soc Med Nat Iasi. 2001, 105(1), 169-71" the method of DE 2714713 is presented and compared with a method that uses nicotinic acid ethyl ester as starting product (7)

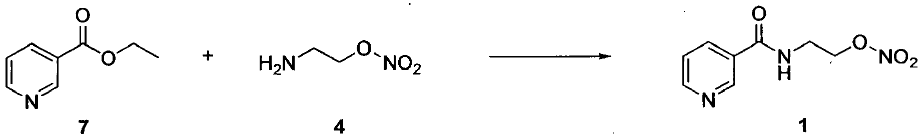

ES 534942 discloses the preparation of Nicorandil by treating 3-pyridinecarboxylic acid hydrazide **(8)** with 2-aminoethyl nitrate (4) in the presence of HgO

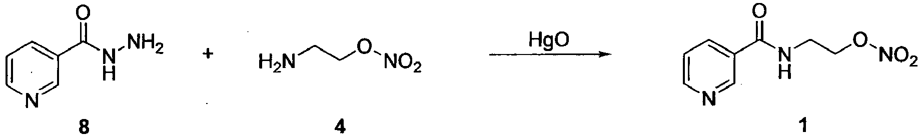

ES 549223 discloses a procedure for synthesising Nicorandil by reacting the nicotinic acid imidazolide derivative **(9)** with 2-aminoethyl nitrate **(4)**

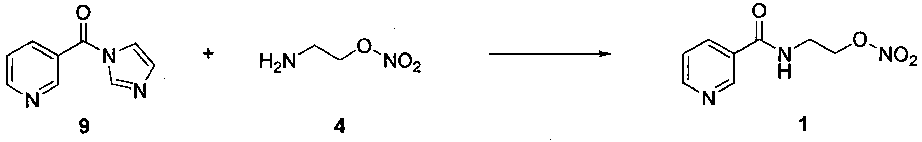

In all these synthesis methods, 2-aminoethyl nitrate or a salt thereof is used as starting product. The industrial synthesis of 2-aminoethyl nitrate is extremely risky in terms of safety, because low molecular weight nitro esters are known to involve high risks connected with their explosive potential, as confirmed by the severe restrictions governing the transport of these substances.

Another synthesis route, extensively described in the literature, uses silver nitrate to perform a nucleophilic substitution on a compound of general formula **(10),** wherein Y is typically a halogen atom

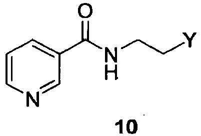

ES 548692 applies the method starting from the iodo-derivative **(11)** 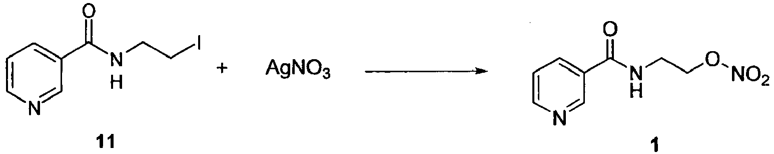 while ES 543857 starts from the chloro-derivative **(12)**

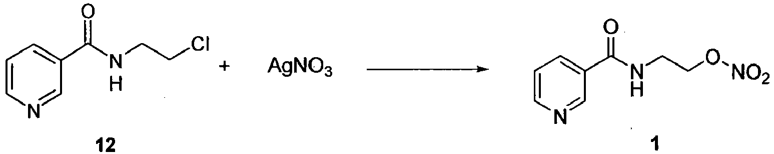

Synthesis methods using silver salts are not very suitable for industrial application due to their high cost and the particular procedures required to treat the reaction mixtures and process wastewater.

JP 61015847 discloses the possibility of performing a nucleophilic substitution (formally with nitrate ion) on a compound of general formula **10,** where Y = MeSO₃ **(13)** in the presence of a phase-transfer catalyst such as tetrabutylammonium nitrate **(14),** using dioxane at 90°C as solvent.

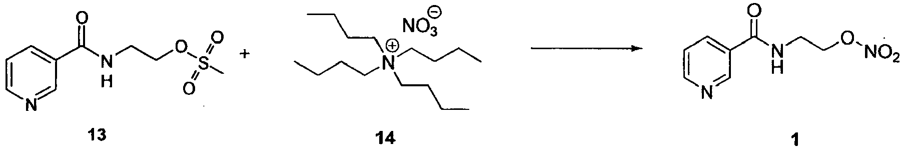

The compounds of formula 10 are unstable at room temperature due to the presence of the leaving group, very easily producing elimination byproducts.

The method of synthesising Nicorandil from intermediates of formula **12** or the like is also disadvantageous as it intrinsically involves one more synthesis step than the traditional routes.

Numerous references cite the possibility of direct nitration of the oxygen of N-(2-hydroxyethyl)nicotinamide **(15)**

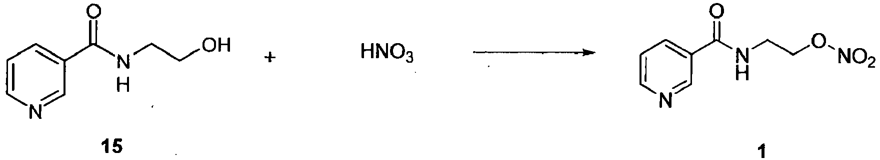

The preparation of **15** is extensively described, for example in Chem. Ber 87, 1628-31 (1954*),* ES 536328, ES 542899, and is simple to perform.

The nitrating agents most commonly used to conduct such nitration are fuming nitric acid used in the absence of other solvents (ES 531337, ES 542899, *"*Yiyao Gongye (1983), (11), 2-3", JP 02207072, PL 162496), fuming nitric acid in the presence of a chlorinated solvent (RO 114613), or a nitrating mixture consisting of nitric and sulphuric acid (ES 531337, ES 536328, RU 2147577 and RU 2341517), also in the presence of small amounts of water (<15%).

A method that identifies nitration conditions with a sulphuric acid/nitric acid mixture in the presence of water, which potentially presents no detonation risk, is further discussed in *"*Russian Journal of Applied Chemistry (2009), 82(10), 1776-1779"*.* The repetition of the synthesis methods proposed (comparative examples 1, 2 and 3 below) demonstrates that if the process described in the literature is performed exactly in accordance with the conditions indicated, the yield and quality data reported (comparative example 1) will be reproduced, but in the event of even minor variations in temperature (comparative example 2), which may occur during the scale-up of the process due to inadequate dimensioning of the thermostating system or to a malfunction of the apparatus, or variations in the residence time of the reaction mass (comparative example 3), potentially dangerous degradation phenomena can be triggered.

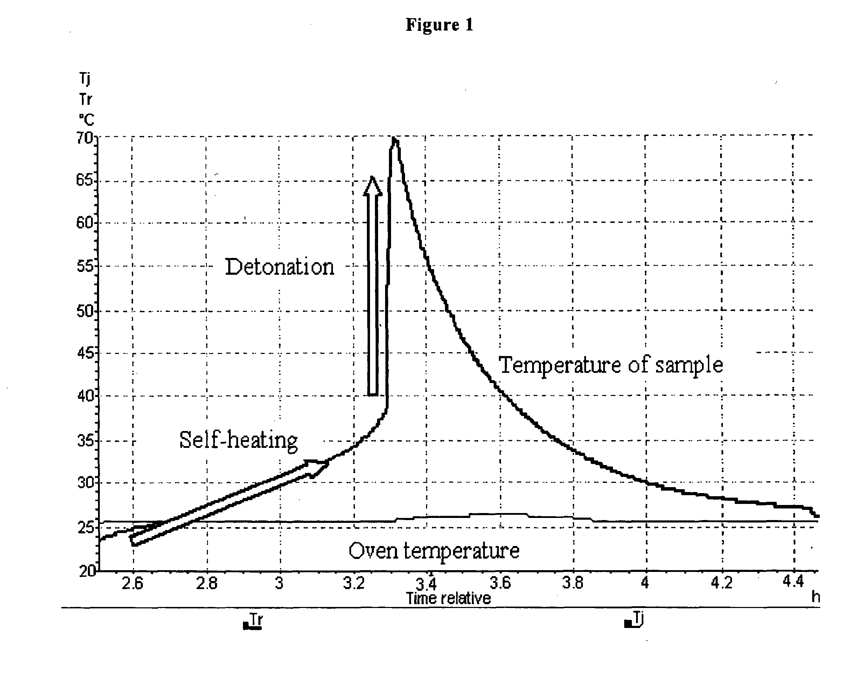

A more accurate study of the thermal stability of the final reaction mass, conducted using the SYSTAG RADEX calorimeter, for a Nicorandil manufacturing process with hydrated sulphuric acid/nitric acid mixture as indicated in *"*Russian Journal of Applied Chemistry (2009), 82(10), 1776-1779*"* and reverified in comparative example 1, indicates a potentially explosive degradation phenomenon (Figure 1).

Although the proposed reaction conditions (low temperatures and strict thermostating) allow a good result to be obtained in terms of yield and quality of the product isolated, the margins of tolerance for a dangerous process are too narrow for the process to be described as safe.

In general, all the methods that use nitric acid (fuming, in sulphuric acid/nitric acid mixture or in the presence of other solvents) are subject to these limitations. One reason may be the use in those methods of a significant stoichiometric excess of nitric acid, which is useful to shift the reaction towards the formation of the nitro-derivative due to the mass effect (obtaining acceptable conversions), but very harmful because it increases the possibility of collateral oxidation reactions. Even the precaution of working at temperatures well below room temperature may not be sufficient to control the process; in the event of malfunction of the cooling system, the temperature of the mass could increase, with the risk of triggering dangerous reactions.

Safe industrial application of these methods therefore requires plants which are appropriately dimensioned in terms of thermostating performance, equipped with redundant control or rapid quenching systems (although as quenching systems are usually based on immediate dilution of the out-of-control reaction mass, they would need very large management volumes, requiring the adoption of small industrial batches and in practice prejudicing the productivity of the processes).

The Nicorandil synthesis processes described in the literature are therefore disadvantageous in economic and industrial safety terms; there is consequently a need for a process with characteristics of economic efficiency and safety at the same time.

### DESCRIPTION OF THE INVENTION

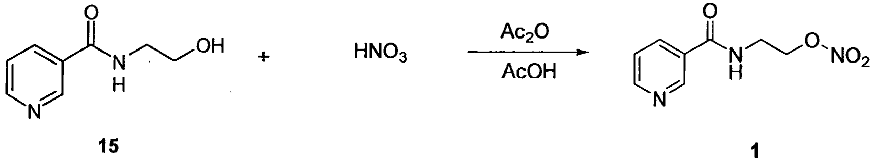

A novel process for the preparation of Nicorandil starting from N-(2-hydroxyethyl)nicotinamide (15) has now been found, wherein the O-nitration reaction is conducted with a nitrating mixture consisting of nitric acid, acetic acid and acetic anhydride. The reaction is preferably carried out at the temperature of 10-30°C, preferably 22°C, in a semi-batch procedure, adding intermediate 15 to the nitrating mixture consisting of nitric acid, acetic acid and acetic anhydride. Said nitrating mixture is prepared from a solution of 68% nitric acid in acetic acid, dropwise adding the acetic anhydride, and maintaining the system at the temperature of 10-30°C, preferably 15-20°C; the reaction is exothermic and fast, and the emission of heat is consequently controllable by regulating the addition rate.

For each weight part of 68% HNO₃, 4-10 parts of acetic acid (preferably 4.5) and 2-5 parts of acetic anhydride (preferably 3) are used to prepare the nitrating mixture. Oxidation inhibitors like the one described in ES 531337, which absorb the nitrogen oxides present in the system, are not necessary, as in 68% nitric acid they are virtually absent. Said nitrating mixture is heat-stable at the temperatures specified for its production.

The molar stoichiometric ratio of HNO₃ to the substrate to be nitrated (15) used is 2-2.4, preferably 2.2, but as nitric acid is the acid species with the lowest pKa in the reaction environment, it must be borne in mind that 1 eq will be consumed to give the nitrate salt of **(15).** The actual stoichiometric excess of HNO₃, depending on the nitration reaction, will therefore amount to 0-40% (20% in the preferred case). The excess nitric acid used in the process according to the invention is much less than that needed to conduct the reaction with other nitrating systems (sulphuric acid/nitric acid mixture or fuming nitric acid), thus reducing the possibility of triggering dangerous oxidation reactions.

By adding the substrate to be nitrated over a period of 15-150 minutes (preferably 45 minutes) to the nitrating mixture in the system, stirred and thermostated at the temperature indicated, the desired product is obtained with an almost quantitative conversion of intermediate **15.** As the reaction is fast and exothermic, the heat emission can be regulated by adjusting the timing of the addition.

The main by-product of the reaction is 2-(nicotinamido)ethyl acetate **(16)** which, under the reaction conditions described, is present in the reaction mixture in a concentration below 2%.

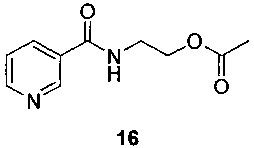

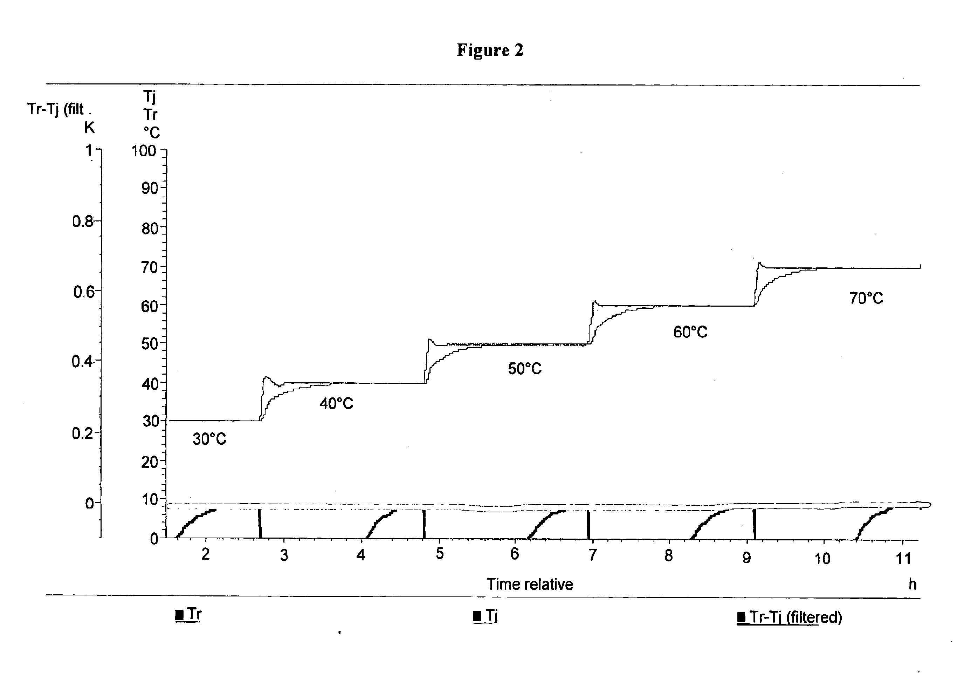

The final reaction mixture, characterised in terms of heat stability with the SYSTAG RADEX calorimeter (Figure 2), did not show any thermal phenomena in the temperature range analysed (30-70°C).

The reaction mixture is poured into a 24% ammonia aqueous solution, using an amount such that the pH is basic at the end of the addition.

The crude Nicorandil is isolated by filtration after thermostating the heterogeneous mixture at the temperature of 0-15°C (preferably 5°C). The solid isolated is recrystallised from water and dried under vacuum at 40°C for 48 hours. The product obtained is analysed with the HPLC technique using a Kinetex HILIC column and 0.1M formate/acetonitrile buffer as eluent phase. The purity of the solid obtained is > 99.5%.

The isolation yield, starting from intermediate **15,** exceeds 90%.

The Nicorandil synthesis process is more advantageous than the known processes because as well as solving the problem of the instability of the reaction mixture, with the consequent potential risk of explosions, it is also characterised by a higher yield.

### DESCRIPTION OF FIGURES

**Figure 1****:** The sample of the reaction mixture, loaded into a vessel (glass-steel) and inserted into an oven set at a constant temperature (25°C), showed a significant self-heating phenomenon followed by very severe decomposition (defined in the diagram as "detonation" in view of the rate of temperature increase associated with it). If the temperature values found are corrected according to the thermal inertia of the system, the result is a thermal phenomenon which would increase the temperature of the sample by approx. 438K; however, the measurement conducted underestimates the event, because it is not conducted under adiabatic conditions but by maintaining the surroundings of the sample at room temperature.
**Figure 2****:** The sample was analysed using a glass-steel vessel and a heating programme of 10K/2h from 30°C to 70°C. The resulting thermogram can be interpreted similarly to a DSC thermogram on a time scale. The exothermic phenomena give a positive effect (EXO UP).

The invention will be described in greater detail in the examples below.

### Example 1

### Synthesis of Nicorandil

6.00 g of glacial acetic acid, followed by 1.35 g of 68% nitric acid, is charged into a 100 mL three-necked reaction flask purged with nitrogen and equipped with an anchor magnetic stirrer, temperature probe and dropping funnel. The system is thermostated at the temperature of 22°C, and 4.00 g of acetic anhydride is added in approx. 15 minutes. The system is stirred for approx. 15 minutes; 1.00 g of N-(2-hydroxyethyl)nicotinamide is then added, in portions, in ≈15 minutes, without exceeding 22°C. The system is stirred for 30 minutes at 22°C, and the progress of the reaction is then checked by HPLC. The N-(2-hydroxyethyl)nicotinamide content is < 1%. The contents of the flask are poured slowly into 24% aqueous ammonia (14.0 g), taking care not to exceed the temperature of 35°C. The temperature is reduced to 5°C and maintained for approx. 30 minutes. The solid is isolated by filtration, washing it with 1 ml of water.

The solid is dried at 40°C for 48 hours, to obtain 1.13 g of crude Nicorandil (yield 89.1 %).

### Example 2

### Preliminary scale-up

300 g of glacial acetic acid, followed by 67.5 g of 68% nitric acid, is charged into a 1000 mL three-necked reaction flask purged with nitrogen and equipped with anchor magnetic stirrer, temperature probe and dropping funnel. The system is thermostated at the temperature of 18°C, and 200 g of acetic anhydride is added in approx. 45 minutes, taking care not to exceed the temperature of 25°C and stopping the addition if that threshold is exceeded. The reaction is very fast, and is therefore controllable by means of the reagent feed. The system is stirred for approx. 30 minutes, then cooled to 15°C, and 50.0 g of N-(2-hydroxyethyl)nicotinamide is added in five identical 10.0 g portions, 15 minutes apart, taking care to return the temperature to 15°C before adding each portion. The system is stirred for 90 minutes at 22°C, and the progress of the reaction is then checked by HPLC. The N-(2-hydroxyethyl)nicotinamide content is < 1%. The contents of the flask are poured slowly into 24% aqueous ammonia (700 g), taking care not to exceed the temperature of 35°C. The temperature is reduced to 5°C and maintained for approx. 60 minutes. The solid is isolated by filtration, washing it with ≈50 ml of water.

The solid is dried at 40°C for 16 hours, and 59.3 g of crude Nicorandil is obtained (yield 93.8%).

### Comparative example 1

### Synthesis from sulphuric acid/nitric acid mixture (reaction time 90 minutes; temperature 10°C)

18.4 g of concentrated sulphuric acid is charged into a 100 mL three-necked reaction flask purged with nitrogen and equipped with anchor magnetic stirrer, temperature probe and dropping funnel. The mixture is cooled to 0°C and 16.9 g of 68% nitric acid is then dropped therein, operating carefully in view of the strong exothermic reaction. The system is thermostated at the temperature of 10°C and 10.0 g of N-(2-hydroxyethyl)nicotinamide is added in approx. 5 minutes, maintaining said temperature. The system is stirred for 30 minutes, and the conversion is then tested by HPLC. The residual N-(2-hydroxyethyl)nicotinamide content is 33.5%. The reaction mixture is colourless. The mixture is left under stirring at 10°C for a further hour, and the conversion is then checked again. The residual N-(2-hydroxyethyl)nicotinamide content is 25.3%. The reaction mixture has turned a yellow-brown colour. The contents of the flask are poured slowly into 24% aqueous ammonia (45.6 g), taking care not to exceed the temperature of 35°C. The temperature is reduced to 5°C and maintained for approx. 30 minutes. The solid is isolated by filtration, washing it with 10 ml of water.

The solid is dried at 40°C for 24 hours, and 7.6 g of crude Nicorandil is obtained (yield 60.2%).

### Comparative example 2

### Synthesis from sulphuric acid/nitric acid mixture (reaction time 90 minutes; temperature 20°C)

18.4 g of concentrated sulphuric acid is charged into a 100 Ml three-necked reaction flask purged with nitrogen and equipped with anchor magnetic stirrer, temperature probe and dropping funnel. The mixture is cooled to 0°C and 16.9 g of 68% nitric acid is then dropped therein, operating carefully in view of the strong exothermic reaction. The system is thermostated at the temperature of 20°C and 10.0 g of N-(2-hydroxyethyl)nicotinamide is added in approx. 5 minutes, maintaining said temperature. The system is stirred for 30 minutes. An evident emission of nitrous vapours is observed at this point, and the mass turns brown. The test is stopped.

### Comparative example 3

### Synthesis from sulphuric acid/nitric acid mixture (reaction time 150 minutes; temperature 10°C)

18.4 g of concentrated sulphuric acid is charged into a 100 mL three-necked reaction flask purged with nitrogen and equipped with anchor magnetic stirrer, temperature probe and dropping funnel. The mixture is cooled to 0°C and 16.9 g of 68% nitric acid is then dropped therein, operating carefully in view of the strong exothermic reaction. The system is thermostated at the temperature of 10°C, and 10.0 g of N-(2-hydroxyethyl)nicotinamide is added in approx. 5 minutes, maintaining said temperature. The system is stirred for 30 minutes, and the conversion is then tested by HPLC. The residual N-(2-hydroxyethyl)nicotinamide content is 31.5%. The reaction mixture is colourless. The mixture is left under stirring at 10°C for a further hour, and the conversion is then checked again. The residual N-(2-hydroxyethyl)nicotinamide content is 25.0%. The reaction mixture will have turned a yellow-brown colour.

If the reaction mass is left at 10°C for a further hour, the formation of brown nitrous vapours will be observed. The residual N-(2-hydroxyethyl)nicotinamide content is 23.8%. The test is stopped.

## Claims

1. A process for the preparation of Nicorandil, which comprises the O-nitration of N-(2-hydroxyethyl)nicotinamide with a mixture of non-fuming nitric acid, acetic acid and acetic anhydride.

2. A process as claimed in claim 1 wherein the reaction temperature ranges from 10 to 30°C.

3. A process as claimed in claim 2 wherein the reaction is carried out at 22°C, in semi-batch mode, adding N-(2-hydroxyethyl)nicotinamide on the mixture of nitric acid, acetic acid and acetic anhydride.

4. A process as claimed in one or more of claims 1 to 3 wherein the nitrating mixture consists of one part of 68% nitric acid, 4-10 parts of acetic acid and 2-5 parts of acetic anhydride.

5. A process as claimed in one or more of claims 1 to 4 wherein the molar ratio of nitric acid to N-(2-hydroxyethyl)nicotinamide is 2-2.4.

## Patentansprüche

1. Verfahren zur Herstellung von Nicorandil, welches die O-nitrierung von N-(2-Hydroxyethyl)nicotinamid mit einem Gemisch aus nicht-rauchender Salpetersäure, Essigsäure und Essigsäureanhydrid umfasst.

2. Verwahren wie in Anspruch 1 beansprucht, wobei die Reaktionstemperatur von 10 bis 30 °C reicht.

3. Verfahren wie in Anspruch 2 beansprucht, wobei die Reaktion bei 22 °C in einer halbkontinuierlichen Verfahrensweise durch Zugabe von N-(2-Hydroxyethyl)nicotinamid zur Mischung von Salpetersäure, Essigsäure und Essigsäureanhydrid durchgeführt wird.

4. Verfahren wie in einem oder mehreren der Ansprüche 1 bis 3 beansprucht, wobei die Nitrierungsmischung aus einem Teil 68 %iger Salpetersäure, 4 bis 10 Teilen Essigsäure und 2 bis 5 Teilen Essigsäureanhydrid besteht.

5. Verfahren wie in einem oder mehreren der Ansprüche 1 bis 4 beansprucht, wobei das molare Verhältnis von Salpetersäure zu N-(2-Hydroxyethyl)nicotinamid 2 bis 2,4 beträgt.

## Revendications

1. Procédé pour la préparation du nicorandil, qui comprend la nitration O du N-(2-hydroxyéthyl)nicotinamide avec un mélange d'acide nitrique non fumant, d'acide acétique et d'anhydride acétique.

2. Procédé selon la revendication 1, dans lequel la température réactionnelle se situe dans la plage de 10 à 30 °C.

3. Procédé selon la revendication 2, dans lequel la réaction est mise en oeuvre à 22 °C, dans un mode en semi-discontinu, en ajoutant du N-(2-hydroxyéthyl)nicotinamide sur le mélange d'acide nitrique, d'acide acétique et d'anhydride acétique.

4. Procédé selon une ou plusieurs des revendications 1 à 3, dans lequel le mélange de nitration est constitué par une partie d'acide nitrique à 68 %, de 4 à 10 parties d'acide acétique et de 2 à 5 parties d'anhydride acétique.

5. Procédé selon une ou plusieurs des revendications 1 à 4, dans lequel le rapport molaire de l'acide nitrique au N-(2-hydroxyéthyl)nicotinamide s'élève de 2 à 2,4.
